# EUROPÄISCHE PATENTSCHRIFT

(11) **EP 2 097 748 B1**
(45) Veröffentlichungstag und Bekanntmachung des Hinweises auf die Patenterteilung: **08.08.2012**
(21) Anmeldenummer: 07866202.0
(22) Anmeldetag: 08.11.2007
(51) Int. Cl.: G01N 33/68, G01N 33/66, G01N 33/74, G01N 33/50

(54) **DIAGNOSE UND RISIKOSTRATIFIZIERUNG VON DIABETES MELLITUS MITTELS MR-PROADM**
DIAGNOSIS AND RISK ASSESSMENT OF PANCREATIC DIABETES USING MR-PROADM
DIAGNOSTIC ET STRATIFICATION DES RISQUES DE DIABÈTE SUCRÉ PAR MR-PROADM

(30) Priorität: 08.11.2006 DE 102006052916
(43) Veröffentlichungstag der Anmeldung: 09.09.2009
(73) Patentinhaber: B.R.A.H.M.S GmbH, 16761 Hennigsdorf (DE)
(72) Erfinder: BERGMANN, Andreas, 12351 Berlin (DE); MORGENTHALER, Nils, 13503 Berlin (DE); PAPASSOTIRIOU, Jana, 14163 Berlin (DE); STRUCK, Joachim, 13465 Berlin (DE)
(74) Vertreter: Simandi, Claus
(86) Internationale Anmeldenummer: PCT/DE2007/002018
(87) Internationale Veröffentlichungsnummer: WO 2008/055491

(56) Entgegenhaltungen:
- WO-A-97/07214
- MORGENTHALER ET AL.: "Measuremnet of midregional proadrenomedullin in plasma with an immunoluminometric assay" CLINICAL CHEMISTRY, Bd. 51, Nr. 10, 2005, Seiten 1823-1829, XP002480767
- BELTOWSKI, J.; JAMROT, A.: "Adrenomeullin - what do we know 10 years since its discovery" POLISH JOURNAL OF PHARMACOLOGY, Bd. 56, 2004, Seiten 5-27, XP002480768
- ITO SHINJI ET AL: "Elevated adrenomedullin in the vitreous of patients with diabetic retinopathy" OPHTHALMOLOGICA, KARGER, BASEL, CH, Bd. 217, Nr. 1, 1. Januar 2003 (2003-01-01), Seiten 53-57, XP009100223 ISSN: 0030-3755

## Beschreibung

Die Erfindung betrifft ein Verfahren zur Diagnose und / oder Risikostratifizierung von Diabetes mellitus, insbesondere von diabetischen Folgeerkrankungen, wobei eine Bestimmung des Markers midregionalem proAdrenomedullin (MR-proADM: SEQ ID No. 2) oder ein Teilpeptid oder Fragment davon oder enthaltend in einer Markerkombination (Panel, Cluster) an einem zu untersuchenden Patienten durchgeführt wird. Weiterhin betrifft die Erfindung die Verwendung eine diagnostischen Vorrichtung und eines Kits zur Durchführung des Verfahrens.

Die Diagnose von Diabetes mellitus ist für Adrenomedullin bekannt (Garcia-Unzueta MT, Montalban C, Pesquera C, Berrazueta JR, Amado JA. Plasma adrenomedullin levels in type 1 diabetes. Relationship with clinical parameters. Diabetes Care. 21:999-1003, 1998 und Turk HM, Buyukberber S, Sevinc A, Ak G, Ates M, Sari R, Savli H, Cigli A. Relationship between plasma adrenomedullin levels and metabolic control, risk factors, and diabetic microangiopathy in patients with type 2 diabetes.Diabetes Care 23:864-7, 2000). Nachteilig ist jedoch, dass aufgrund der mangelnden Stabilität des Adrenomedullins sowie dessen Kurzweiligkeit (Lewis LK, Smith MW, Yandle TG, Richards AM, Nicholls MG. Adrenomedullin (1-52) measured in human plasma by radioimmunoassay: plasma concentration, adsorption, and storage. Clin Chem. 44:571-7 1998) im Plasma keine sichere Diagnose erfolgen kann.

Es besteht jedoch ein Bedürfnis für Diabetes mellitus eine sichere Diagnose zu stellen oder eine (Risiko)Stratifizierung vorzunehmen, insbesondere hinsichtlich weiterer klinischen Entscheidungen und insbesondere hinsichtlich des Schweregrades einer Diabetes mellitus oder diabetischen Folgeerkrankungen.

Ferner ist im Stand der Technik die proAdrenomedullin (proADM)-Bestimmung in der Diagnose beschrieben (EP0622458B1), insbesondere zwecks Untersuchung von Sepsis (EP1121600B1). Zudem ist ein weiteres Fragment des proAdrenomedullins - nämlich das sogenannte midregionalem proAdrenomedullin (MR-proADM: SEQ ID No. 2, auch: AS45-92 der preproADM in SEQ ID No. 1 (Figur 1)) in EP1488209B1 zu diagnostischen Zwecken offenbart. Die Eignung des midregionalen proAdrenomedullin (MR-pröADM: SEQ ID No. 2) zur Diagnose von Diabetes mellitus wird jedoch nicht offenbart.

Es ist Aufgabe der vorliegenden Erfindung ein verbessertes Verfahren zur Diagnose und / oder Risikostratifizierung von Diabetes mellitus, insbesondere von diabetischen Folgeerkrankungen, bereitzustellen.

Die Aufgabe wird durch ein Verfahren zur in-vitro Diagnose und / oder Risikostratifizierung von Diabetes mellitus gelöst, wobei eine Bestimmung des Markers midregionalen proAdrenomedullin (MR-proADM: SEQ ID No. 2) oder ein Teilpeptid oder Fragment davon oder enthaltend in einer Markerkombination (Panel, Cluster) an einem zu untersuchenden Patienten durchgeführt wird (nachstehend erfindungsgemäßes Verfahren).

Der Begriff "Risikostratifizierung" umfasst erfindungsgemäß das Auffinden von Diabetespatienten, insbesondere solche mit diabetischen Folgeerkrankungen, mit der schlechteren Prognose, zwecks intensiverer Diagnostik und (Folge-) Therapie/Behandlung von Diabetes mellitus mit dem Ziel einen möglichst günstigen Verlauf der Diabetes mellitus zu ermöglichen.

Besonders vorteilhaft kann daher mittels des erfindungsgemäßen Verfahren eine sichere Diagnose und / oder Risikostratifizierung erfolgen. Das erfindungsgemäße Verfahren ermöglicht klinische Entscheidungen, die zu einer schnelleren Diagnose, insbesondere der diabetischen Folgeerkrankungen, führen. Solche klinische Entscheidungen umfassen ebenfalls weiterführende Behandlung mittels Arzneimitteln zur Behandlung oder Therapie von Diabetes mellitus.

In einer weiteren bevorzugten Ausführungsform des erfindungsgemäßen Verfahrens erfolgt die Diagnose und / oder Risikostratifizierung zur Prognose, zur Prophylaxe, zur differentialdiagnostischen Früherkennung und Erkennung, zur Beurteilung des Schweregrades und zur therapiebegleitenden Verlaufsbeurteilung von Diabetes mellitus.

In einer weiteren Ausführungsform des erfindungsgemäßen Verfahrens wird dem zu untersuchenden Patienten Körperflüssigkeit, insbesondere Blut entnommen, wahlweise Vollblut, Serum oder erhältliches Plasma und die Diagnose erfolgt *in vitro*/*ex vivo,* d.h. außerhalb des menschlichen oder tierischen Körpers. Aufgrund der Bestimmung des Markers midregionalem proAdrenomedullin (MR-proADM: SEQ ID No. 2) oder Teilpeptide oder Fragmente davon und seiner vorhandenen Menge bzw. dessen Mengenveränderung gegenüber einer Referenz in mindestens einer Patientenprobe kann die Diagnose und / oder Risikostratifizierung erfolgen.

Im Rahmen dieser Erfindung wird unter "Diabetes mellitus", insbesondere der Typ II Diabetes mellitus (Insulinresistenz) verstanden, eine chronische Stoffwechselerkrankung, wobei die Insulinproduktion in den β-Zellen der Langerhansschen Inseln in der Bauchspeicheldrüse gestört ist oder Insulin zwar vorhanden ist, an seinem.Zielort, den Zellmembranen, aber nicht richtig wirken kann. Die Folge dieser gestörten Insulinpröduktion bzw. -wirkung sind erhöhte Blutzuckerwerte (Hyperglykämie). Beim diabetischen Krankheitsverlauf unterscheidet man zwischen Prädiabetes, bei dem es erst in seinem Endstadium zu einer laborchemisch nachweisbaren "gestörten Glukosetoleranz" kommt, und dem eigentlichen manifesten Diabetes mellitus. Am Anfang der prädiabetischen Krankheitsphase steht die Insulinresistenz. Nahezu zeitgleich entwickeln sich bereits eine endotheliale Dysfunktion, Hyperlipoproteinämie und hypertensive Kreislaufdysregulation. Die Folge dieser Risikokonstellation sind zudem arteriosklerotische Gefäßwandveränderungen (Mikro- und Makroangiopathie) sowie vaskuläre Komplikationen infolge Mikrozirkulationsstörungen. Weitere Folge- Begleiterkränkungen sind diabetische Retinopathie bis zur Erblindung, sowie die Nephropathie bis hin zur Niereninsuffizienz, die Neuropathie, das diabetische Fußsyndrom und kardiovaskuläre Komplikationen.

Daher betrifft die Erfindung die Diagnose und / oder Risikostratifizierung der Diabetes mellitus vom Typ II und dessen Folge- und Begleiterkrankungen, insbesondere endotheliale Dysfunktionen, Hyperlipoproteinämie, hypertensive Kreislaufdysregulation, diabetische Retinopathie, Nephropathie, Niereninsuffizienz, Neuropathie, diabetische Fußsyndrom und kardiovaskuläre Komplikationen.

Alle genannten Indikationen werden zudem z.B. im Pschyrembel, De Gruyter, Berlin 2004 beschrieben.

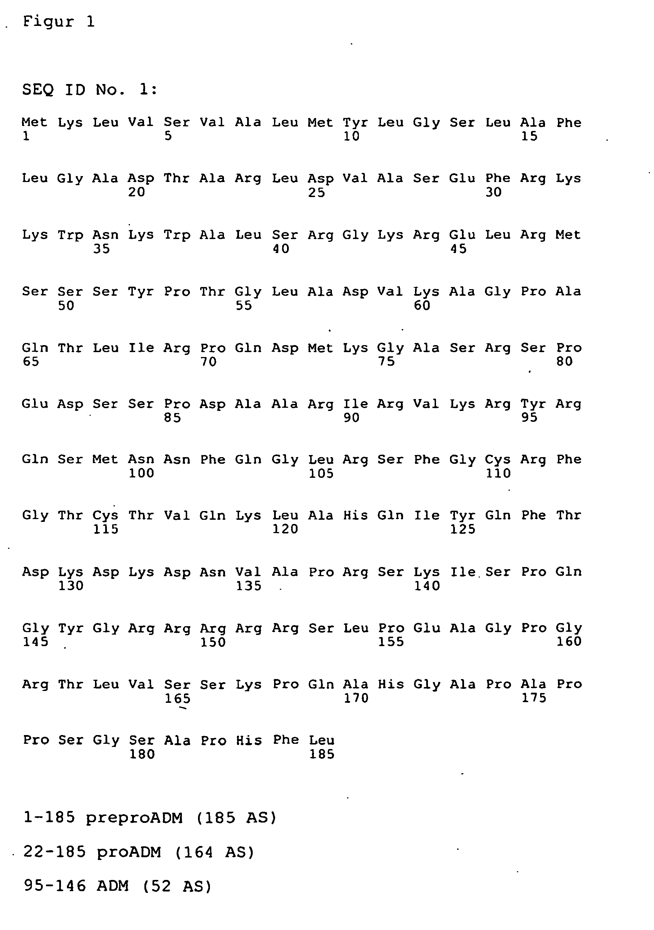

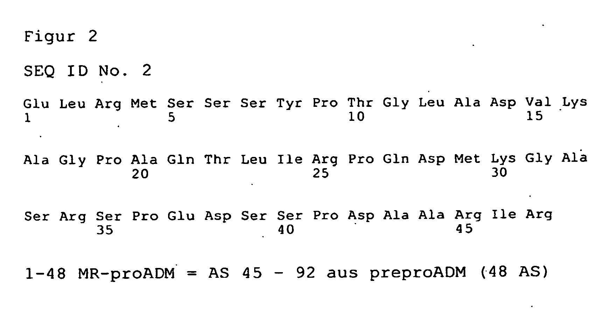

Im Rahmen dieser Erfindung wird unter "midregionalem proAdrenomedullin (MR-proADM: SEQ ID No. 2)" ein humanes Protein oder Polypeptid verstanden mit einer Aminosäuresequenz von 45-92 (Position 45 ist Glu, Position 92 ist Val) der SEQ ID No. 1 (Figur 1) des preproAdrenomedullins (Kitamura K, Sakata J, Kangawa K, Kojima M, Matsuo H, Eto T. Cloning and characterization of cDNA encoding a precursor for human adrenomedullin Biochem Biophys Res Commun 1993;194:720-725) bzw. Aminosäuresequenz 1-48 der SEQ ID No. 2 (Figur 2). Dieses Fragment des proAdenomedullins wird "midregionales proAdrenomedullin (MR-proADM)" bezeichnet (EP 1488209B1) und weist besonders vorteilhaft eine hohe Plasmastabilität auf.

Ferner kann das erfindungsgemäße "midregionale proAdrenomedullin" Modifikationen aufweisen, wie Glykolisierung, Lip(o)idisierung oder Derivatisierungen.

In einer weiteren Ausführungsform kann die Bestimmung von midregionalem proAdrenomedullin (MR-proADM: SEQ ID No. 2) zusätzlich mit weiteren Markern erfolgen, wobei das midregionale proAdrenomedullin (MR-proADM: SEQ ID No. 2) in einer Markerkombination (Panel, Cluster) enthalten ist, und zwar vorzugsweise solche, die bereits auf eine Diabetes mellitus hinweisen. In einer weiteren besonderen Ausführungsform kann dies ebenfalls ein vaskulärer Marker sein, der auf eine Diabetes-begleitende endotheliale Dysfunktion des Kreislaufs hinweisen kann.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, vaskulärer Marker an einem zu untersuchenden Patienten durchgeführt wird.

Erfindungsgemäß wird der inflammatorische Marker aus mindestens einem Marker aus der Gruppe C-reaktivem Protein (CRP), Cytokinen, wie etwa TNF-alpha, Interleukinen, wie etwa IL-6, Interleukin-1β, Procalcitonin (1-116, 3-116), Angiotensin II, Endothelin-1 und Adhäsionsmolekülen, wie VCAM oder ICAM ausgewählt sowie der vaskuläre Marker aus mindestens einem Marker aus der Gruppe Kreatinkinase, Myeloperoxidase, Myoglobin, natriuretisches Protein, insbesondere ANP (bzw. ANF), proANP, NT-proANP, BNP, proBNP, NT-proBNP oder jeweils eine Teilsequenz davon, CRP ausgewählt . Ferner werden hierunter vorzugsweise Kreislaufregulierende (Pro)Hormone verstanden, insbesondere wie pro-Gastrin-Releasing Peptid (proGRP), pro-Endothelin-1, pro-Leptin, pro-Neuropeptid-Y, pro-Somatostatin, pro-Neuropeptid-YY, pro-Opiomelanocortin oder jeweils eine Teilsequenz davon.

In einer weiteren Ausführungsform kann zusätzlich mindestens ein diabetischer Marker/Faktor bestimmt werden. Diabetische Marker/Faktoren sind erfindungsgemäß insbesondere solche wie Adiponectin, Kohlenhydrate, Fette, wie Cholesterine (LDH) u.a., Bodymass Index (BMI), Alter, Blutdruck, HOMA-IR (Homeostasis Model Assessment-Insulin Resitance index, zur Bestimmung siehe: Matthews DR, Hosker JP, Rudenski AS, Naylor BA, Treacher DF, Turner RC, Homeostasis Model Assessment: Insulin Resistence and B-cell Function from Fasting Plasma Glucose and Insulin Concentrations in Man. Diabetologia 28: 412-419. 1985).

In einer weiteren Ausführungsform der Erfindung kann das erfindungsgemäße Verfahren mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen an einem Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), sei es in Einzel- oder Multiparameterbestimmung.

Ferner betrifft die Erfindung die Verwendung von midregionalem proAdrenomedullin (MR-proADM: SEQ ID No. 2) oder Teilpeptide oder Fragmente davon oder enthaltend in einer Markerkombination (Panel, Cluster) zur in-vitro Diagnose und / oder Risikostratifizierung von Diabetes mellitus, insbesondere Diabetes mellitus vom Typ II und dessen Folge- und Begleiterkrankungen, sowie insbesondere unter Berücksichtigung der oben genannten Ausführungsformen. Die Markerkombination kann ggfs. einen weiteren geeigneten Marken enthalten.

Eine weitere Aufgabe ist die Verwendung einer diagnostischen Vorrichtung zur Durchführung der erfindungsgemäßen Verfahren.

Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung des erfindungsgemäßen Verfahrens.

Die Erfindung betrifft zudem die Verwendung eines Kit zur in-vitro Diagnose und Risikostratifizierung von Diabetes mellitus, insbesondere Diabetes mellitus vom Typ II und dessen Folge- und Begleiterkrankungen, wobei eine Bestimmung von midregionalem proAdrenomedullin (MR-proADM: SEQ ID No. 2) oder Teilpeptide oder Fragmente davon oder enthaltend in einer Markerkombination (Panel, Cluster) an einem zu untersuchenden Patienten durchgeführt wird, insbesondere unter Berücksichtigung der oben genannten Ausführungsformen. Solche Nachweisreagenzien umfassen z.B. Antikörper, etc.

Nachfolgende Beispiele und Figuren dienen zur näheren Erläuterung der Erfindung, jedoch ohne die Erfindung auf diese Beispiele und Figuren zu beschränken.

### Beispiele:

### Beispiel 1:

Der MR-proADM Assay wurde nach Morgenthaler et al. (Morgenthaler NG, Struck J, Alonso C, Bergmann A. Measurement of midregional proadrenomedullin in plasma with an immunoluminometric assay. Clin Chem. 2005 Oct; 51(10):1823-9) durchgeführt.

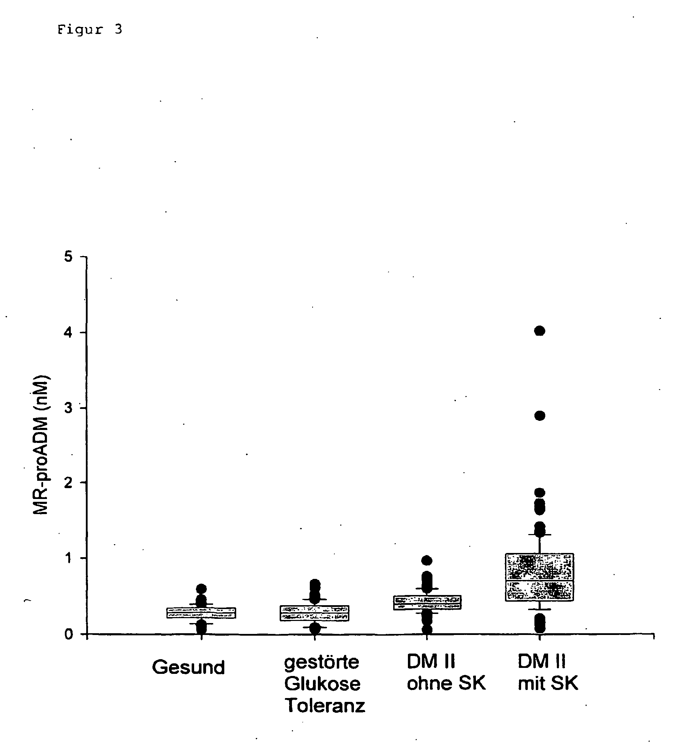

MR-proADM wurde in 100 gesunden Probanden mit ungestörter Glukosetoleranz, 60 Patienten mit bereits gestörter Glukosetoleranz und 200 Patienten mit manifestiertem Diabetes mellitus Typ II (kurz: "DM II") bestimmt. Diese 200 Patienten unterteilten sich in 100 Diabetiker ohne Folgeerkrankungen und 100 Diabetiker mit bereits vorhandenen Folgeerkrankungen, wie diabetische Nephropathie und diabetische Retinopathie. Figur 3 zeigt einen signifikanten Anstieg der MR-proADM Werte mit zunehmendem Schweregrad des DM II. Insbesondere die beiden Gruppen von DM II Patienten unterschieden sich von den gesunden Kontrollen und den Patienten mit gestörter Glucosetoleranz. Überraschenderweise fanden sich die höchsten MR-proADM Werte in den DM II Patienten, die bereits diabetische Folgeerkrankungen zeigten. Tabelle 1 zeigt neben MR-proADM die Diabetes relevanten Parameter wie Glucose und HbA1c in den jeweiligen Gruppen.

**Tabelle 1 (Spätkomplikationen (SK) (syn: Folgeerkrankungen)):**

| | Gesund (n=100) | Gestörte Glukose Toleranz (n=60) | DM II Ohne SK (n=100) | DM II Mit SK (N=100) | *p* value (for trend) |
|---|---|---|---|---|---|
| Geschlecht (% male) | 50 | 32 | 58 | 55 | 0.007 |
| Alter (yr) | 40 (14) | 44 (11) | 58 (10) | 61 (10) | <0.001 |
| Dauer der Erkrankung (Jahre) | - | - | 16(7) | 17 (9) | 0.55 |
| Body mass index (kg/m²) | 22.9 (3.7) | 24.1 (4.2) | 25.8 (4.2) | 26.1 (4.5) | <0.001 |
| SystotischerBlutdruck (mmHg) | 121 (14) | 126 (22) | 136 (17) | 147 (21) | <0.001 |
| Diastolischer Blutdruck (mmHg) | 77 (8) | 78 (12) | 81 (8) | 81(11) | 0.02 |
| Nüchtern Plasma Glukose (mmol/L) | 4.9 (0.3) | 5.9 (0.4) | 7.0 (1.2) | 8.5 (3.5) | <0.001 |
| HBA1c (%) | - | - | 7.8(1.3) | 8.1(1.6) | 0.07 |
| Midregionales-proadrenomedullin (mmol/L) | 0.27 (0.09) | 0.29 (0.13) | 0.42 (0.13) | 0.81(0.54) | <0.001 |

Der Zusammenhang zwischen bereits vorhandener eingeschränkter Mikrozirkulation und MR-proADM Werten zeigt sich auch durch eine signifikante Korrelation (r = 0.43, P = 0,002) zwischen den MR-proADM Werten bei 50 Patienten mit DM II und dem Zirkulationsfluss gemessen als resting forearm cutaneous micro-circulatory perfusion. (RCMP: A Enrique Caballero, Rola Saouaf, Subodh Arora, Su C Lim, Frank W. LoGerfo, Edward S Horton, Aristidis Veves. Reactivity of the micro- and macrocirculation is impaired in those at risk for type 2 diabetes. Diabetes 48:1856-1862, 1999).

### Figuren:

Figur 1 zeigt die SEQ ID No. 1 des preproADM samt zugehörige Teilsequenzen.
Figur 2 zeigt SEQ ID No. 2 des MR-proADM
Figur 3 zeigt MR-proADM in gesunden Probanden, Patienten mit gestörter Glucosetoleranz, Patienten mit Diabetes mellitus Typ II (DM II) ohne Spätkomplikationen (SK) (syn: Folgeerkrankungen) und Patienten mit DM II mit Spätkomplikationen (syn: Folgeerkrankungen).

## Patentansprüche

1. Verfahren zur in-vitro Diagnose und/oder Risikostratifizierung von Diabetes mellitus,
**dadurch gekennzeichnet,**
**dass** eine Bestimmung von midregionalem proAdrenomedullin (MR-proADM: SEQ ID No. 2) in einer Probe eines zu untersuchenden Patienten durchgeführt wird.

2. Verfahren nach Anspruch 1,
**dadurch gekennzeichnet,**
**dass** die in-vitro Diagnose und/oder Risikostratifizierung von Diabetes mellitus vom Typ II und dessen Folge- und Begleiterkrankungen, insbesondere endotheliale Dysfunktionen, Hyperlipoproteinämie, hypertensive Kreislaufdysregulation, diabetische Retinopathie, Nephropathie, Niereninsuffizienz, Neuropathie, diabetische Fußsyndrom und kardiovaskuläre Komplikationen erfolgt

3. Verfahren nach einem der vorherigen Ansprüche,
**dadurch gekennzeichnet,**
**dass** zusätzlich eine Bestimmung mindestens eines weiteren Marker ausgewählt aus der Gruppe inflammatorischer Marker,
und zwar aus mindestens einem Marker aus der Gruppe C-reaktivem Protein (CRP), Cytokinen, wie etwa TNF-alpha, Interleukinen, wie etwa IL-6, Interleukin-1ß, Procalcitonin (1-116, 3-116), Angiotensin II, Endothelin-1 ausgewählt ist und / oder
einem vaskulären Marker und zwar aus mindestens einem Marker aus der Gruppe Kreatinkinase, Myeloperoxidase, Myoglobin, natriuretisches Protein, insbesondere ANP (bzw. ANF), proANP, NT-proANP, BNP, proBNP, NT-proBNP oder Krelslaufregulierende (Pro)Hormone wie pro-Gastrin-Releasing Peptid (proGRP), pro-Endothelin-1, pro-Leptin, pro-Neuropeptid-Y, pro-Somatostatin, pro- Neuropeptid-YY, pro-Opiomelanocortin ausgewählt ist und / oder diabetischer Marker/Faktor von einem zu untersuchenden Patienten durchgeführt wird.

4. Verfahren nach einem der vorherigen Ansprüche,
**dadurch gekennzeichnet,**
**dass** parallele oder simultane Bestimmungen der Marker durchgeführt werden.

5. Verfahren nach einem der vorherigen Ansprüche,
**dadurch gekennzeichnet,**
**dass** die Bestimmungen mittels einer Patientenprobe durchgeführt werden.

6. Verfahren nach einem der vorherigen Ansprüche,
**dadurch gekennzeichnet,**
**dass** die Bestimmungen mittels einem Schnelltest, insbesondere in Einzel- oder Multiparameterbestimmungen durchgeführt werden.

7. Verfahren nach einem der vorherigen Ansprüche, **dadurch gekennzeichnet, dass** die Diagnose zur Stratifizierung von Patienten für klinische Entscheidungen, insbesondere weiterführende Behandlung mittels Arzneimitteln zur Behandlung oder Therapie von Diabetes mellitus, insbesondere Diabetes mellitus vom Typ II und dessen Folge- und Begleiterkrankungen erfolgt.

8. Verfahren nach einem der vorherigen Ansprüche,
**dadurch gekennzeichnet,**
**dass** die Diagnose und / oder Risikostratifizierung zur Prognose, zur Prophylaxe, zur differentialdiagnostischen Früherkennung und Erkennung, zur Beurteilung des Schweregrades und zur therapiebegleitenden Verlaufsbeurteilung von Diabetes mellitus, insbesondere Diabetes mellitus vom Typ II und dessen Folge- und Begleiterkrankungen erfolgt.

9. Verwendung von midregionalem proAdrenomedullin (MR-proADM: SEQ ID No. 2) oder enthaltend in einer Markerkombination zur in-vitro Diagnose und / oder Risikostratifizierung von Diabetes mellitus, insbesondere Diabetes mellitus vom Typ II und dessen Folge- und Begleiterkrankungen, wobei die Markerkombination weitere Marker nach Anspruch 3 enthält oder ggfs. einen weiteren geeigneten Marker.

10. Verwendung einer diagnostischen Vorrichtung zur Durchführung eines Verfahrens nach einem der Ansprüche 1 bis 8.

11. Verwendung eines Kits zur in-vitro Diagnose und / oder Risikostratiflzierung von Diabetes mellitus, insbesondere Diabetes mellitus vom Typ II und dessen Folge- und Begleiterkrankungen,
enthaltend Nachweisreagenzien zur Bestimmung des Markers midregionalem proAdrenomedullin (MR-proADM: SEQ ID No. 2) oder enthaltend in einer Markerkombination, wobei die Markerkombination weitere Marker nach Anspruch 3 enthält und Hilfsmittel zur Durchführung eines Verfahrens nach einem der Ansprüche 1 bis 8.

## Claims

1. Method for in vitro diagnosis and/or risk stratification of diabetes mellitus,
**characterized in that**
a determination of midregional proadrenomedullin (MR-proADM: SEQ ID No. 2) is carried out in a sample of a patient to be investigated.

2. Method according to claim 1,
**characterized in that** in vitro diagnosis and/or risk stratification of Type II diabetes mellitus and its sequelae and concomitant illnesses, particularly endothelial dysfunction, hyperlipoproteinemia, hypertensive dysregulation of the cardiovascular system, diabetic retinopathy, nephropathy, renal insufficiency, neuropathy, diabetic foot syndrome, and cardiovascular complications, take place.

3. Method according to one of the preceding claims,
**characterized in that**
a determination is additionally carried out with at least one further marker selected from the group of inflammatory markers,
namely at least one marker selected from the group of C-reactive protein (CRP), cytokines, such as TNF-alpha, for example, interleukins, such as IL-6, interleukin-1ß, procalcitonin (1-116, 3-116), angiotensin II, endothelin-1,
or vascular markers, namely at least one marker selected from the group of creatine kinase, myeloperoxidase, myoglobin, natriuretic protein, particularly ANP (or ANF), proANP, NT-proANP, BNP, proBNP, NT-proBNP, or (pro)hormones that regulate the cardiovascular system, such as pro-gastrin-releasing peptide (proGRP), pro-endothelin-1, pro-leptin, pro-neuropeptide-Y, pro-somatostatin, pro-neuropeptide-YY, pro-opiomelanocortin,
and/or diabetic markers/factors, in a patient to be investigated.

4. Method according to one of the preceding claims,
**characterized in that**
parallel or simultaneous determinations of the markers are carried out.

5. Method according to one of the preceding claims,
**characterized in that**
the determinations are carried out on at least one patient sample.

6. Method according to one of the preceding claims,
**characterized in that** the determinations are carried out by means of a rapid test, particularly with single-parameter or multi-parameter determinations.

7. Method according to one of the preceding claims,
**characterized in that** the diagnosis takes place for the stratification of patients for clinical decisions, particularly further treatment by means of medications for the treatment or therapy of diabetes mellitus, particularly Type II diabetes mellitus, and its concomitant illnesses and sequelae.

8. Method according to one of the preceding claims,
**characterized in that** the diagnosis and/or risk stratification takes place for prognosis, for prophylaxis, for early detection and detection by means of differential diagnosis, for assessment of the degree of severity, and for assessing the course of diabetes mellitus, particularly Type II diabetes mellitus, and its concomitant illnesses and sequelae, as an accompaniment to therapy.

9. Use of midregional proadrenomedullin (MR-proADM: SEQ ID No. 2), or embraced in a marker combination, for in vitro diagnosis and/or risk stratification of diabetes mellitus, particularly Type II diabetes mellitus, and its concomitant illnesses and sequelae, wherein the marker combination contains other markers according to claim 3, or another suitable marker, if necessary.

10. Use of a diagnostic device for carrying out a method according to one of claims 1 to 8.

11. Use of a kit for in vitro diagnosis and/or risk stratification of diabetes mellitus, particularly Type II diabetes mellitus, and its concomitant illnesses and sequelae,
comprising detection reagents for determining the marker midregional proadrenomedullin (MR-proADM: SEQ ID No. 2), or embraced in a marker combination, wherein the marker combination contains other markers according to claims 3, and ancillary substances for carrying out a method according to one of claims 1 to 8.

## Revendications

1. Méthode pour le diagnostic et/ou la stratification du risque in vitro du diabète sucré, **caractérisée par le fait qu'**une détermination de la proadrénomédulline de région médiane (MR-proADM : SEQ ID N° 2) est réalisée dans un échantillon d'un patient à étudier.

2. Méthode selon la revendication 1, **caractérisée par le fait que** le diagnostic et/ou la stratification du risque in vitro du diabète sucré de Type II et ses séquelles et maladies concomitantes, en particulier le dysfonctionnement endothélial, l'hyperlipoprotéinémie, la dérégulation hypertensive du système cardiovasculaire, la rétinopathie diabétique, la néphropathie, l'insuffisance rénale, la neuropathie, le syndrome du pied diabétique et des complications cardiovasculaires, ont lieu.

3. Méthode selon l'une des revendications précédentes, **caractérisée par le fait qu'**une détermination est additionnellement mise en oeuvre avec au moins un autre marqueur choisi dans le groupe des marqueurs inflammatoires, à savoir au moins un marqueur choisi dans le groupe de : la protéine C-réactive (CRP), des cytokines, telles que TNF-alpha, par exemple, des interleukines, telles que IL-6, l'interleukine-1β, la procalcitonine (1-116, 3-116), l'angiotensine II, l'endothéline-1, ou des marqueurs vasculaires, à savoir au moins un marqueur choisi dans le groupe de : la créatine kinase, les myéloperoxydases, la myoglobine, la protéine natriurétique, en particulier ANP (ou ANF), proANP, NT-proANP, BNP, proBNP, NT-proBNP, ou des (pro)hormones qui régulent le système cardiovasculaire, telles que le peptide de libération de la progastrine (proGRP), la pro-endothéline-1, la pro-leptine, le pro-neuropeptide Y, la prosomatostatine, le pro-neuropeptide YY, la proopiomélanocortine et/ou des marqueurs/facteurs diabétiques, chez un patient à étudier.

4. Méthode selon l'une des revendications précédentes, **caractérisée par le fait que** des déterminations parallèles ou simultanées des marqueurs sont mises en oeuvre.

5. Méthode selon l'une des revendications précédentes, **caractérisée par le fait que** les déterminations sont mises en oeuvre au moins dans un échantillon de patient.

6. Méthode selon l'une des revendications précédentes, **caractérisée par le fait que** les déterminations sont mises en oeuvre au moyen d'un test rapide, en particulier des déterminations à paramètre unique ou à paramètres multiples.

7. Méthode selon l'une des revendications précédentes, **caractérisée par le fait que** le diagnostic a lieu pour la stratification de patients pour des décisions cliniques, en particulier un traitement supplémentaire au moyen de médications pour le traitement ou la thérapie du diabète sucré, en particulier du diabète sucré de Type II, et ses maladies et séquelles concomitantes.

8. Méthode selon l'une des revendications précédentes, **caractérisée par le fait que** le diagnostic et/ou la stratification du risque a lieu pour le pronostic, pour la prophylaxie, pour la détection précoce et la détection au moyen d'un diagnostic différentiel, pour l'estimation du degré de sévérité, et pour l'estimation du déroulement du diabète sucré, en particulier du diabète sucré de Type II, et de ses maladies et séquelles concomitantes, en tant qu'accompagnement de la thérapie.

9. Utilisation de la proadrénomédulline de région médiane (MR-proADM : SEQ ID No 2), ou intégrée dans une combinaison de marqueurs, pour un diagnostic et/ou une stratification du risque in vitro du diabète sucré, en particulier du diabète sucré de Type II, et ses maladies et séquelles concomitantes, dans lesquelles la combinaison de marqueurs contient d'autres marqueurs selon la revendication 3, ou un autre marqueur adapté, si nécessaire.

10. Utilisation d'un dispositif de diagnostic pour la mise en oeuvre d'une méthode selon l'une des revendications 1 à 8.

11. Utilisation d'une trousse pour le diagnostic et/ou la stratification du risque in vitro du diabète sucré, en particulier du diabète sucré de Type II, et de ses maladies et séquelles concomitantes, comprenant des réactifs de détection pour la détermination du marqueur pro-adrénomédulline de région médiane (MR-proADM : SEQ ID N° 2), ou intégrée dans une combinaison de marqueurs, la combinaison de marqueurs contenant d'autres marqueurs selon la revendication 3, et des substances auxilliaires pour la mise en oeuvre d'une méthode selon l'une des revendications 1 à 8.


### SEQUENCE LISTING

<110> Brahms Aktiengesellschaft
<120> Diagnose und Risikostratifizierung von Diabetes mellitus mittels MR-proADM
<130> BRAHMS 0029WO 06/11
<140> PCT/DE2007 not yet known
<141> 2007-11-08
<160> 2
<170> PatentIn version 3.3
<210> 1
   <211> 185
   <212> PRT
   <213> Human
<400> 1
   Met Lys Leu Val Ser Val Ala Leu Met Tyr Leu Gly Ser Leu Ala Phe
   1 5 10 15
   Leu Gly Ala Asp Thr Ala Arg Leu Asp Val Ala Ser Glu Phe Arg Lys
   20 25 30
   Lys Trp Asn Lys Trp Ala Leu Ser Arg Gly Lys Arg Glu Leu Arg Met
   35 40 45
   Ser Ser Ser Tyr Pro Thr Gly Leu Ala Asp Val Lys Ala Gly Pro Ala
   50 55 60
   Gln Thr Leu Ile Arg Pro Gln Asp Met Lys Gly Ala Ser Arg Ser Pro
   65 70 75 80
   Glu Asp Ser Ser Pro Asp Ala Ala Arg Ile Arg Val Lys Arg Tyr Arg
   85 90 95
   Gln Ser Met Asn Asn Phe Gln Gly Leu Arg Ser Phe Gly Cys Arg Phe
   100 105 110
   Gly Thr Cys Thr Val Gln Lys Leu Ala His Gln Ile Tyr Gln Phe Thr
   115 120 125
   Asp Lys Asp Lys Asp Asn Val Ala Pro Arg Ser Lys Ile Ser Pro Gln
   130 135 140
   Gly Tyr Gly Arg Arg Arg Arg Arg Ser Leu Pro Glu Ala Gly Pro Gly
   145 150 155 160
   Arg Thr Leu Val Ser Ser Lys Pro Gln Ala His Gly Ala Pro Ala Pro
   165 170 175
   Pro Ser Gly Ser Ala Pro His Phe Leu
   180 185
<210> 2
   <211> 47
   <212> PRT
   <213> Human
<400> 2
   Glu Leu Arg Met Ser Ser Ser Tyr Pro Thr Gly Leu Ala Asp Val Lys
   1 5 10 15
   Ala Gly Pro Ala Gln Thr Leu Ile Arg Pro Gln Asp Met Lys Gly Ala
   20 25 30
   Ser Arg Ser Pro Glu Asp Ser Ser Pro Asp Ala Ala Arg Ile Arg
   35 40 45